# FASCICULE DE BREVET EUROPEEN

(11) **EP 0 426 567 B1**
(45) Date de publication et mention de la délivrance du brevet: **10.05.1995**
(21) Numéro de dépôt: 90403086.3
(22) Date de dépôt: 31.10.1990
(51) Int. Cl.: A61B 5/04, A61B 5/0436

(54) **Analyseur d'électrocardiogramme de longue durée**
Analysegerät eines Langzeit-Elektrokardiogramms
Device for analysing a long-time electrocardiogram

(30) Priorité: 02.11.1989 FR 8914352
(43) Date de publication de la demande: 08.05.1991
(73) Titulaire: ELA MEDICAL, F-92120 Montrouge (FR)
(72) Inventeur: Faisandier, Yves, F-75014 Paris (FR)
(74) Mandataire: Loyer, Bertrand

(56) Documents cités:
- GB-A- 1 446 643
- GB-A- 2 181 554
- US-A- 3 909 792
- US-A- 4 098 267

## Description

La présente invention concerne le domaine médical et plus précisément l'analyse de l'activité cardiaque d'un patient.

Il est connu par le document US-A-3 909 792 de suivre l'électrocardiogramme (ECG) d'un patient, sur un écran qui en représente 4 heures d'enregistrement, et d'effectuer une mémorisation de 4 Secondes d'enregistrement lors de la détection d'un battement prématuré.

Afin de connaître de manière plus complète l'activité cardiaque d'un patient, il est connu de procéder à un enregistrement en ambulatoire pendant une durée de 24 heures, ou même plus longue, de différents paramètres physiologiques du patient et principalement de son électrocardiogramme (ECG). Cette analyse par la méthode dite de Holter tend à largement se développer mais reste actuellement mal exploitée.

En effet, si l'enregistrement des données est fait de manière connue et fiable, l'analyse des données ECG n'est pas encore satisfaisante.

Tout d'abord, les analyseurs performants sont très coûteux et les cardiologues n'ont pas tous la possibilité d'en posséder un personnellement.

Les médecins sont alors obligés d'envoyer les enregistrements à des centres de traitement, ce qui d'une part, allonge les délais et d'autre part leur enlève toute possibilité d'interprétation personnelle en fonction de la connaissance qu'ils ont du patient.

Un autre inconvénient des analyseurs actuels, réside dans leur mise en oeuvre. Certains sont très rigides d'utilisation et analysent les données enregistrées en suivant un programme strict ne laissant qu'une faible faculté d'intervention à un opérateur. Les résultats donnés par ces analyseurs ne sont pas toujours exploitables, car il ne sont pas très fiables du fait que les signaux d'électrocardiogramme peuvent être très variables d'un sujet à l'autre et que de nombreux artefacts en basse ou haute fréquence peuvent venir perturber l'enregistrement.

D'autres analyseurs laissent une plus grande liberté d'intervention à l'opérateur, mais nécessitent en revanche sa présence continue. Généralement de tels analyseurs mènent leur étude en proposant à l'opérateur de valider l'interprétation des pathologies retenues, car présentant une difficulté.

La présente invention tend alors à proposer un nouvel analyseur permettant au médecin de mener facilement et personnellement l'analyse des données contenues dans un enregistrement. A cet effet, la présente invention a pour objet un analyseur de bande ECG d'électrocardiogramme de longue durée, du type présentant un résultat global de l'analyse sous la forme d'un ensemble de courbes tenant sur au moins un écran, et donnant pour différents paramètres leur évolution sur toute la durée de l'enregistrement, caractérisé en ce qu'il comporte : un premier curseur à déplacement vertical pour sélectionner un paramètre à analyser sur l'écran, un deuxième curseur à déplacement horizontal sur l'échelle des temps, et des moyens pour visualiser , en réponse à une sélection particulière de la position des premier et deuxième curseurs, une portion de l'ECG correspondant à l'instant précis où un événement déterminé par le paramètre sélectionné au moyen du premier curseur a eu lieu pour la première fois après l'instant défini au moyen du deuxième curseur.

Selon d'autres caractéristiques de l'invention :
- l'impression d'un événement comporte une première ligne donnant le contexte de l'événement à échelle réduite et un graphique le reproduisant à grande échelle.
- la représentation sur écran pour un type d'événement est assurée pour une succession d'échantillons placés l'un au dessus de l'autre, les événements de chaque échantillon étant décalés sur l'échelle des temps de façon à donner à l'image représentée un effet tridimensionnel.

La présente invention sera mieux comprise à la lecture de la description qui va suivre en référence aux dessins annexés dans lesquels :
- la figure 1 montre l'affichage disponible en fin d'analyse ;
- les figures 2a, 2b, 2c montrent différentes possibilités d'affichage d'un événement déterminé ;
- la figure 3 montre la représentation d'un événement selectionné lors d'une impression ;
- la figure 4 montre une représentation d'échantillons d'ECG à décalage temporel.

La première originalité de l'analyseur selon l'invention, tient au fait qu'il met en oeuvre des appareils financièrement accessibles à un très grand nombre de personnes puisqu'il s'agit d'un micro-ordinateur associé à une imprimante laser. Ce matériel a l'avantage de pouvoir être utilisé pour d'autres services que l'analyse des électrocardiogrammes, tels que le traitement de textes, la gestion de fichiers,...

Ce matériel est, de plus, d'une maintenance peu coûteuse et l'on peut très facilement modifier les logiciels qu'il contient, pour suivre leur évolution .

L'analyseur selon la présente invention, effectue une analyse automatique ou corrigée par le médecin de l'électrocardiogramme, puis présente un résultat global sous la forme d'un ensemble de courbes, tenant sur un écran.

Le médecin a à sa disposition plusieurs écrans récapitulatifs qu'il peut sélectionner selon l'analyse qu'il veut mener. Ces écrans concernent
- les troubles du rythme,
- les troubles de la repolarisation,
- les modes de fonctionnement d'un stimulateur cardiaque,
- les défauts de fonctionnement d'un stimulateur cardiaque,
- les enregistrements polygraphiques (c'est-à-dire concernant des paramètres autres que l'ECG).

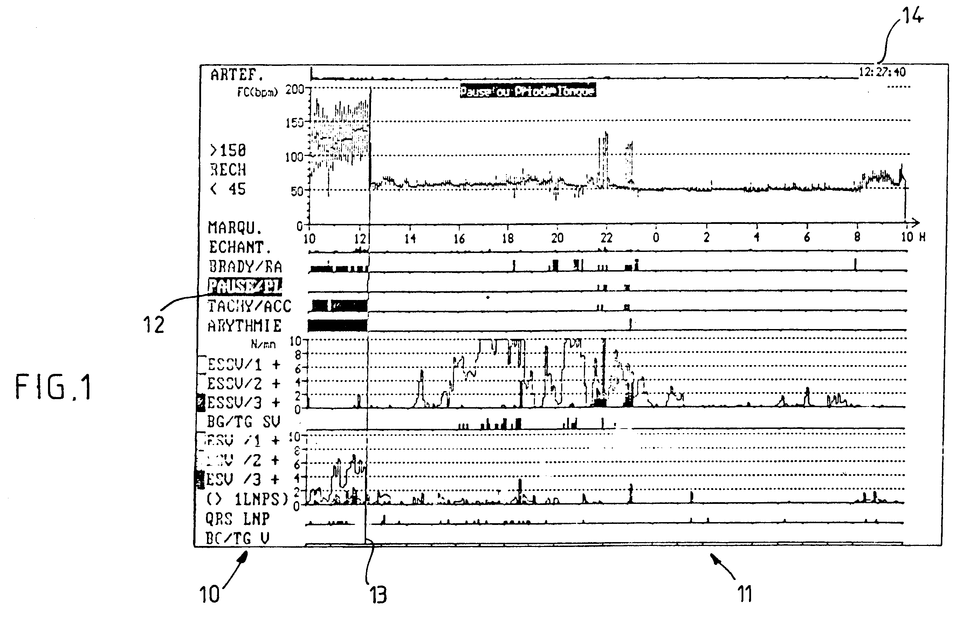

La figure 1 montre l'affichage mis à la disposition d'un médecin désirant étudier les troubles du rythme; les paramètres à sa disposition sont les suivants :
- Artéfact (Artef),
- Fréquence cardiaque (FC),
- Recherche (RECH)
- Marqueur d'événement (MARQU.),
- Echantillon (ECHANT.),
- Bradycardie/Ralentissement (BRADY/RA),
- Pause et Période longue (PAUSE/PL),
- Tachycardie et accélération (TACHY/ACC.),
- Extrasystole supraventriculaire isolée (ESSV/1),
- ESSV groupées par 2 (Doublet) (ESSV/2),
- ESSV groupées par 3 et + (Salve) (ESSV/3+),
- Bigéminisme et Trigéminisme supraventriculaire (BG/TG SV),
- Extrasystole Ventriculaire isolée (ESV/1),
- ESV groupées par 2 (Doublet) (ESV/2),
- ESV groupées par 3 et + (Salve) (ESV/3+),
- QRS large non prématuré (LNP),
- Bigéminisme et Trigéminisme ventriculaire (BG/TG V).

La partie gauche 10 de cet affichage, récapitule les différents paramètres examinés et la partie droite 11, présente pour chacun desdits paramètres, une courbe donnant leur évolution sur toute la durée de l'enregistrement.

Certains des paramètres proposés sont associés à des valeurs de limites hautes et/ou basses, que le médecin peut à tout moment modifier à sa convenance.

Le médecin peut alors procéder à des analyses plus précises de l'EGC ; à cet effet, il a à sa disposition deux curseurs déplaçables. Le premier curseur 12 que l'on voit ici sur la pause peut être déplacé verticalement afin de sélectionner un paramètre tandis que le deuxième curseur 13, sous la forme d'un trait vertical s'étendant sur toute la hauteur de l'écran, peut être déplacé horizontalement et représente l'heure.

Un affichage 14 donne à tout moment l'heure exacte définie par ledit curseur 13.

Le médecin peut mener son étude d'une première manière en positionnant le curseur 12 sur un paramètre et la machine lui propose alors d'examiner l'ECG à l'instant précis auquel l'événement a eu lieu pour la première fois après un temps de référence défini par la position du curseur 13.

Une deuxième manière de mener l'étude consiste à définir par le curseur 13 l'heure précise de l'ECG que l'on veut examiner.

Le médecin peut alors choisir la durée d'ECG qu'il veut visualiser afin de connaître le contexte de l'événement.

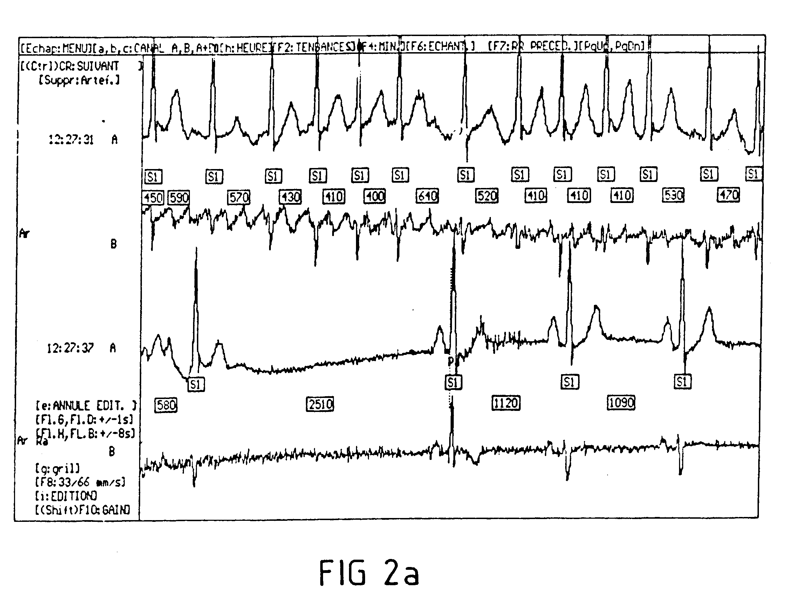

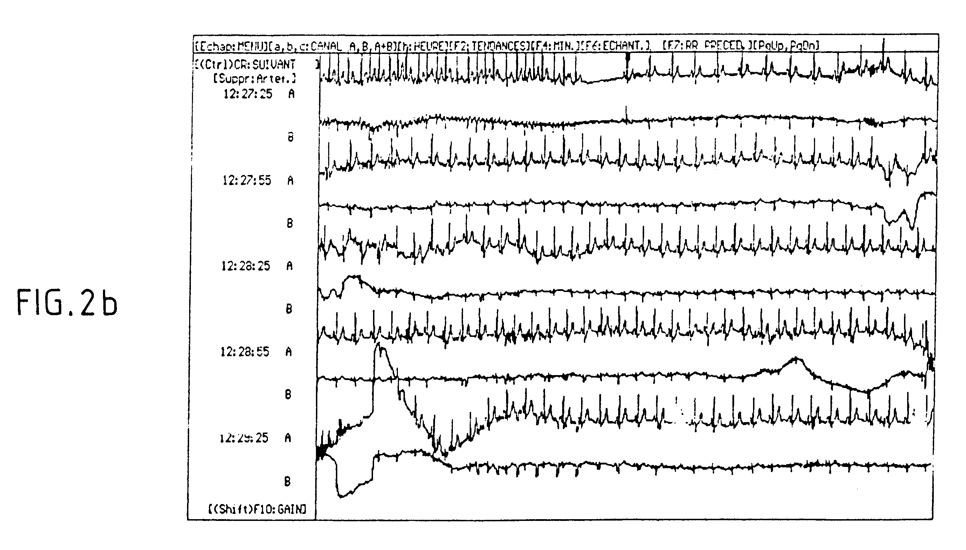

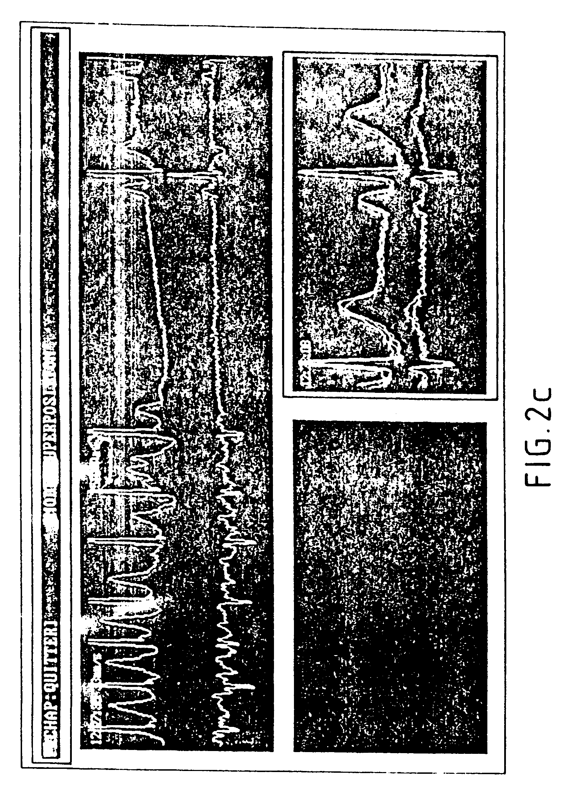

On a représenté à la figure 2 trois formes de visualisation. La figure 2a représente l'écran de visualisation de 12 secondes d'ECG ; l'écran de la figure 2b permet de visualiser 2,5 minutes. La figure 2c représente la méthode la plus classique de visualisation consistant à superposer les courbes afin de voir l'évolution de celles-ci.

Après avoir ainsi examiné un événement, le médecin peut soit rester selon une des représentations de la figure 2 et faire défiler les courbes en fonction du temps, soit retourner à l'écran représenté à la figure 1 pour choisir un autre événement.

Cette alternative est proposée à tout instant ce qui procure au médecin une très grande souplesse lui permettant de mener à bien son analyse.

Dans le cas où il a été demandé un défilement des courbes puis un retour à l'écran de la figure 1 l'heure indiquée en 14 et la position du curseur 13 sont celles auxquelles le défilement est stoppé.

Le médecin peut également à tout moment décider d'avoir ou non l'impression de l'événement.

A cet effet, l'analyseur suivant l'invention propose une présentation originale permettant une meilleure perception de l'événement.

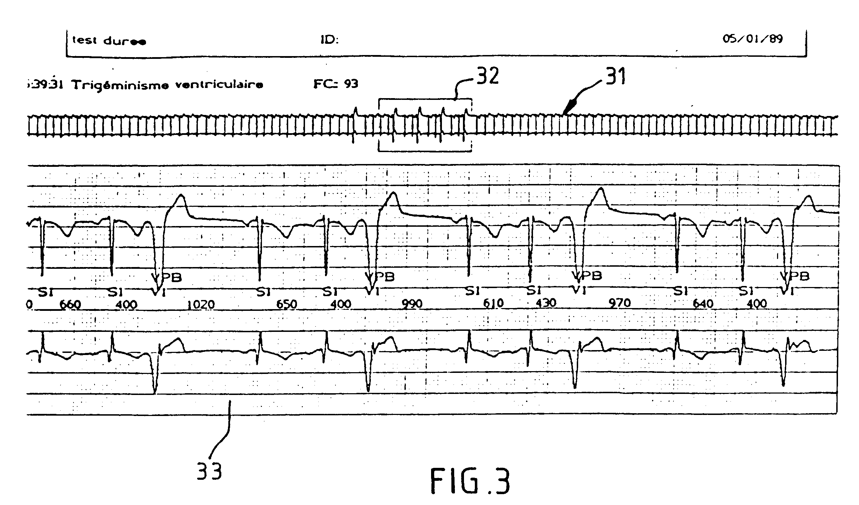

Cette présentation visible à la figure 3, met en parallèle deux représentations de l'événement. Une première ligne 31 reproduit à échelle réduite le contexte de l'événement 32 mis en évidence par un encadrement.

Cette ligne peut, au choix du médecin rappeler 15, 30 ou 60 secondes d'enregistrement. Sous cette première ligne 31, l'événement 32 est reproduit par un graphique 33 à grande échelle. Sur ce graphique 33, une seconde de temps est représentée par 25 mm, le médecin voit alors de manière précise ce qui s'est passé pendant 8 secondes, tout en pouvant d'un simple coup d'oeil à la ligne 31, comprendre et analyser dans quelles conditions s'est passé ledit événement.

Une telle présentation est beaucoup plus facilement utilisable que les présentations imprimées usuel les qui sont soit à échelle réduite regroupant 60 lignes d'une minute par page et nécessitant dans ce cas l'impression de 24 pages pour éditer les 24 heures d'enregistrement, soit à grande échelle (25 ou 50 mm/s) mais ne fournissant alors pas le contexte de survenance des événements.

L'analyseur suivant l'invention permet le passage instantané à tout moment de l'écran total de la figure 1 à l'un des écrans de la figure 2 tout en prévoyant l'impression d'un événement ce qui permet une très grande souplesse d'emploi.

L'analyseur selon l'invention permet également de procéder à une sélection par événement en plaçant le curseur à la manière d'un filtre sur l'événement et en utilisant une représentation à un seul canal pour afficher le signal correspondant.

Le logiciel intervient pour assurer la représentation du deuxième événement avec un léger décalage vers le haut et vers la droite, par exemple, puis celle deg événements suivants avec le même décalage.

De cette manière, il est possible de suivre l'évolution d'un paramètre.

On aura compris qu'avec un tel analyseur, le médecin peut analyser lui-même à sa convenance, la bande rapportée par son malade. Il peut ainsi rechercher les événements représentatifs de l'état du malade et faire imprimer en peu de temps le compte-rendu nécessaire à son analyse et à son suivi dudit malade.

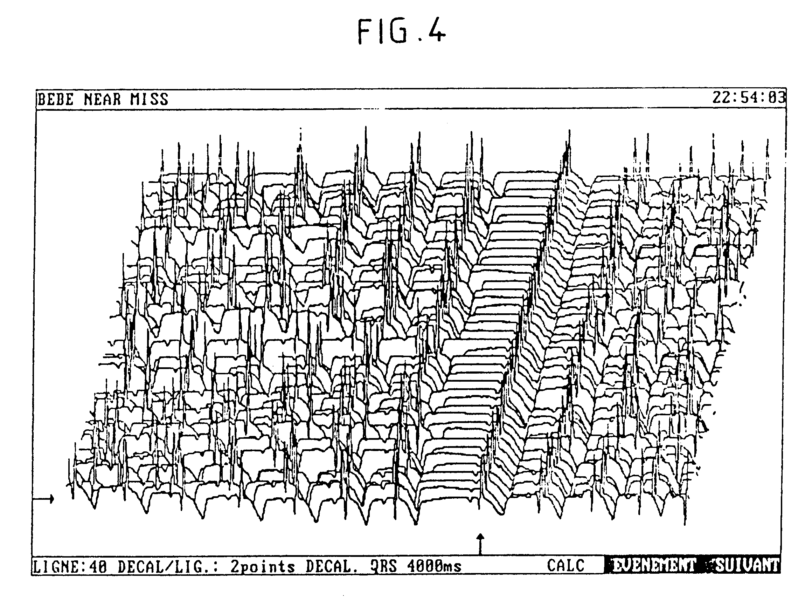

Sur la figure 4 est représentée, selon le mode dit "contourogramme", une succession d'échantillons d'ECG se rapportant à un événement donné. Les échantillons sont placés l'un au dessus de l'autre. L'événement est placé sensiblement en milieu d'écran. Un décalage temporel de 10 à 100 ms entre les événements des échantillon successifs permet de donner un effet tridimensionnel à l'image représentée sur l'écran.

Dans la représentation proposée sur la Figure 4, il est possible de voir plusieurs secondes d'ECG (avant chaque événement, de régler l'échelle des temps, de sélectionner le type d'accident ou d'événement à représenter, de fixer la densité verticale des échantillons (par exemple entre 10 et 80 par éran), d'afficher l'heure de chaque échantillon et d'aller directement d'un des échantillons présent sur l'écran à un autre mode d'affichage.

Selon l'invention, ce mode de représentation permet de visualiser un groupe d'événements reconnus par le système, afin de les valider. Il permet en outre de visualiser le contexte dans lequel surviennent les événements, grâce à la représentation des échantillons qui s'étale sur plusieurs secondes avant l'événement et de prendre en compte, aussi bien des éléments importants (les complexes précédents) que des éléments discrets (les ondes P, T ou U par exemple).

## Revendications

1. Analyseur de bande ECG d'électrocardiogramme de longue durée, du type présentant un résultat global de l'analyse sous la forme d'un ensemble de courbes tenant sur au moins un écran, et donnant pour différents paramètres leur évolution sur toute la durée de l'enregistrement, caractérisé en ce qu'il comporte : un premier curseur (12) à déplacement vertical pour sélectionner un paramètre à analyser sur l'écran, un deuxième curseur (13) à déplacement horizontal sur l'échelle des temps, et des moyens pour visualiser, en réponse à une sélection particulière de la position des premier et deuxième curseurs, une portion de l'ECG correspondant à l'instant précis où un événement déterminé par le paramètre sélectionné au moyen du premier curseur a eu lieu pour la première fois après l'instant défini au moyen du deuxième curseur.

2. Analyseur selon la revendication 1, caractérisé en ce que l'impression d'un événement (32) comporte une première ligne (31) donnant le contexte de l'événement à échelle réduite et un graphique (33) le reproduisant à grande échelle.

3. Analyseur selon l'une des revendications 1 ou 2 caractérisé en ce que la représentation sur écran pour un type d'événement est assurée pour une succession d'échantillons placés l'un au dessus de l'autre, les événements de chaque échantillon étant décalés sur l'échelle des temps de façon à donner à l'image représentée un effet tridimensionnel.

## Claims

1. An ECG-band analyser for a long-duration electrocardiogram, of the type showing a global result of the analysis in the form of a set of curves on at least one screen, and giving for different parameters their evolution over the entire duration of the recording, characterised in that it comprises: a first, vertically displaceable cursor (12) for selecting a parameter to be analyzed on the screen, a second cursor (13) which is horizontally displaceable on the time scale, and means for displaying, in response to a given selection of the position of the first and second cursors, a portion of the ECG corresponding to the precise moment at which an event determined by the parameter selected by means of the first cursor took place for the first time after the moment defined by means of the second cursor.

2. An analyser according to Claim 1, characterised in that the impression of an event (32) comprises a first line (31) giving the context of the event on a reduced scale and a graph (33) reproducing it on a large scale.

3. An analyser according to one of Claims 1 or 2, characterised in that the on-screen representation for a type of event is ensured for a succession of samples placed one above the other, the events of each sample being offset on the time scale so as to give the image shown a three-dimensional effect.

## Patentansprüche

1. Analysegerät des EKG-Streifens eines Langzeit-Elektrokardiogramms, das ein Gesamtergebnis der Analyse in Form einer Kurvenschar darstellt, die auf zumindest einem Bildschirm gehalten wird, und deren Entwicklung während der gesamten Dauer der Aufzeichnung für verschiedene Parameter wiedergibt,
**gekennzeichnet durch**
einen vertikal verschiebbaren ersten Cursor (12) zum Auswählen eines auf dem Bildschirm zu analysierenden Parameters, einen horizontal auf dem Zeitmaßstab verschiebbaren zweiten Cursor (13) und
Einrichtungen, um im Ansprechen auf eine einzelne Auswahl der Position des ersten und zweiten Cursors einen Abschnitt des Elektrokardiogramms sichtbarzumachen, der einem präzisen Zeitpunkt entspricht, in dem ein durch den mittels des ersten Cursors ausgewählten Parameter bestimmtes Ereignis zum ersten Mal nach dem mittels des zweiten Cursors definierten Zeitpunkt aufgetreten ist.

2. Analysegerät nach Anspruch 1,
**dadurch gekennzeichnet, daß**
die Darstellung eines Ereignisses (32) eine erste Linie (31) enthält, die den Zusammenhang des Ereignisses in verkleinertem Maßstab darstellt, und eine Graphik (33), die dieses in großem Maßstab wiedergibt.

3. Analysegerät nach Anspruch 1 oder 2,
**dadurch gekennzeichnet, daß**
die Darstellung auf dem Bildschirm für eine Art des Ereignisses für eine Folge von Meßwerten gesichert ist, die einander überlagert sind, wobei
die Ereignisse aller Meßwerte auf dem Zeitmaßstab derart versetzt sind, daß das wiedergegebene Bild dreidimensional erscheint.
